# EUROPÄISCHE PATENTSCHRIFT

(11) **EP 0 993 433 B1**
(45) Veröffentlichungstag und Bekanntmachung des Hinweises auf die Patenterteilung: **05.02.2003**
(21) Anmeldenummer: 98938682.6
(22) Anmeldetag: 02.07.1998
(51) Int. Cl.: C07C 51/41, C07C 59/19, A61K 31/19

(54) **VERFAHREN ZUR HERSTELLUNG VON CALCIUMPYRUVATEN**
METHOD FOR PRODUCING CALCIUM PYRUVATES
PROCEDE POUR LA PREPARATION DE PYRUVATES DE CALCIUM

(30) Priorität: 11.07.1997 DE 19729786; 21.10.1997 US 955838
(43) Veröffentlichungstag der Anmeldung: 19.04.2000
(73) Patentinhaber: Degussa AG, 83308 Trostberg (DE)
(72) Erfinder: PISCHEL, Ivo, D-83342 Tacherting (DE); WEISS, Stefan, D-83308 Trostberg (DE); ORTENBURGER, Günter, D-83308 Trostberg (DE); KÖNIG, Harro, D-83308 Trostberg (DE)
(74) Vertreter: Weiss, Wolfgang, Dipl.-Chem. Dr.
(86) Internationale Anmeldenummer: EP9804089
(87) Internationale Veröffentlichungsnummer: WO99002479

(56) Entgegenhaltungen:
- EP-A- 0 795 534
- FR-A- 1 465 432
- GB-A- 2 313 544
- US-A- 5 294 641
- US-A- 5 395 822
- US-A- 5 480 909
- US-A- 5 612 374
- US-A- 5 716 926
- US-A- 5 756 469

## Beschreibung

Die vorliegende Erfindung betrifft ein Verfahren zur Herstellung von Calciumpyruvaten, welches sich insbesondere zur Herstellung von sehr reinen und weitgehend wasserfreien Calciumpyruvat-Salzen eignet, neuartige Calciumpyruvat-Salze und deren Verwendung, insbesondere als Bestandteil von physiologisch verträglichen Zusammensetzungen.

Es ist bekannt, daß Salze der Brenztraubensäure (Pyruvate) wertvolle physiologische, therapeutische und dietätische Eigenschaften besitzen. Pyruvate, insbesondere Calciumpyruvate, finden Anwendung zur Steigerung der Ausdauer und der Kraft im Sportbereich, zur Gewichts- und Körperfettreduzierung (U.S. Patent Nr. 4,315,835) sowie als Schutzsubstanz für Körperzellen und Gewebe (besonders für kardiovaskuläres, hepatisches, nephrotisches, peritoneales und neuronales Gewebe) (U.S. Patent Nr. 5, 395,822) und als Substanz zur Inhibition der Radikalbildung (U.S. Patent Nr. 5,480,909) sowie als Radikalfängersubstanz in Körperzellen und Geweben (auch Synovialgewebe), im Gesundheitsbereich und als Nahrungsergänzungsmittel. Des weiteren finden Pyruvate als Wundheilmittel und zur Behandlung von Nierenerkrankungen (akutes Nierenversagen, Nierensteinleiden) Anwendung.

Von den Pyruvatsalzen sind Natrium- und Kaliumpyruvat aufgrund ihres Gehaltes an Natrium- bzw. Kaliumionen für therapeutische Anwendungen und als Nahrungsmittelergänzung jedoch wenig geeignet. Calciumionen führen im Gegensatz zu den Alkalimetallionen zu keinen physiologischen Nebenwirkungen, so daß Calciumpyruvate unbedenklich für die therapeutische Anwendung und als Nahrungsergänzung in Frage kommen.

Entsprechend dem Stand der Technik sind bisher nur zwei Verfahren zur Herstellung von Calciumpyruvaten bekannt geworden. Gemäß der Veröffentlichung von K. Jowanowitsch in Monatshefte 6, 467-476 (1885) wird Weinsäure in Glycerin zu einem Brenztraubensäureglycid dehydratisiert bzw. decarboxyliert, welches anschließend mit Kalk in wäßriger Lösung zum Calciumpyruvat weiterreagiert. Wie bei der Nacharbeitung der Beispiele dieser Veröffentlichung festgestellt werden konnte, entstehen auf diese Weise keine Calciumpyruvate, sondern polymere Brenztraubensäure-Derivate.

Entsprechend der französischen Patentschrift 1 465 432 wird Calciumpyruvat durch Neutralisation von Brenztraubensäure mit Calciumcarbonat,-hydroxid oder -oxid in Wasser neutralisiert. Nachteilig bei diesen Verfahren ist die Tatsache, daß nur unreine und instabile Calciumpyruvate erhalten werden können, die mehr als 2,5 Mol Kristallwasser enthalten und in Form von 2,2-Dihydroxypropionat-Ionen vorliegen. Diese Umsetzungsprodukte weisen in der Regel einen geringen Gehalt an Calciumpyruvat und vergleichsweise große Mengen an Nebenprodukten auf, da die Brenztraubensäure bzw. das Pyruvation durch Aldoladditions- oder Aldolkondensationsreaktionen zur acyclischen oder cyclischen Dimeren und Polymeren der Brenztraubensäure reagiert. Als acyclische Verbindungen sind hierbei insbesondere die Parabrenztraubensäure (4-Hydroxy-4-methyl-2-oxoglutarsäure) und ihre Salze sowie die höheren Aldoladditionsprodukte zu nennen. Des weiteren können Oxal- und Methylbernsteinsäure als Nebenprodukte gebildet werden.

In EP-A-0 795 543 wird ein Verfahren zur Herstellung von Calciumpyruvat-Hydraten durch Umsetzen einer wäßrigen Brenztraubensäure-Lösung mit Calciumcarbonat und Isolieren des beim Ausfällen mit einem organischen Verdünnungsmittel (Aceton) entstandenen Niederschlages an Calciumpyruvat-Hydrat aus der Lösung beschrieben.

Aus den acyclischen Brenztraubensäurepolymeren können wiederum durch Lactonisierung, Ketalisierung, und andere Reaktionen cyclische Verbindungen entstehen, wie z.B. 2-Hydroxy-2-methyl-4-oxoglutarsäure-5-lacton, Trimesinsäure-, Isophthalsäure- und Pyrantricarbonsäurederivate. Diese Nebenprodukte können in gleicher Weise auch bei der Lagerung von Calciumpyruvaten auftreten, die mehr als 2,5 Mol Kristallwasser enthalten.

Die Calciumpyruvate entsprechend dem Stand der Technik sind somit für die Anwendung als Therapeutikum (Radikalfänger, Cytoprotektion, Obesität u.a.) oder als Nahrungsergänzungsmittel nicht geeignet, weil sich bei der Herstellung und Lagerung Neben- und Zersetzungsprodukte der Brenztraubensäure und ihrer Salze bilden, die physiologisch unverträglich oder toxisch sein können.

Der vorliegenden Erfindung lag daher die Aufgabe zugrunde, ein Verfahren zur Herstellung von Calciumpyruvaten zu entwickeln, welches die genannten Nachteile entsprechend dem Stand der Technik nicht aufweist, sondern Calciumpyruvat-Salze mit hoher Reinheit liefert, die zumindest weitgehend frei von toxikologisch bedenklichen Nebenprodukten sind und eine hohe Lagerstabilität aufweisen.

Diese Aufgabe wurde erfindungsgemäß dadurch gelöst, daß man Calciumsalze von organischen Säuren oder aciden organischen Keto- oder Hydroxyverbindungen mit Brenztraubensäure im Temperaturbereich von -20 bis + 120° C gegebenenfalls in Gegenwart eines Verdünnungs- oder Lösemittels umsetzt.

Es hat sich hierbei überraschenderweise gezeigt, daß man auf diese Weise Calciumpyruvate hoher Reinheit herstellen kann, die weitgehend wasserfrei sein können. Außerdem sind die auf diese Weise hergestellten Calciumpyruvate thermisch beständig und besitzen eine sehr gute Lagerstabilität. Dies ist deshalb so überraschend, weil Brenztraubensäure eine relativ instabile Verbindung darstellt und sich bisher bekannte Calciumpyruvate innerhalb kurzer Zeit in dimere und polymere Brenztraubensäure-Derivate zersetzen.

Beim Verfahren entsprechend der vorliegenden Erfindung werden also Calciumsalze von organischen Säuren oder aciden organischen Keto- oder Hydroxy-Verbindungen mit Brenztraubensäure bei Temperaturen von -20 bis +120 °C, vorzugsweise 10 bis 60°C, umgesetzt. Als organische Säuren können beispielsweise aliphatische Monocarbonsäuren eingesetzt werden, die gegebenenfalls noch Substituenten wie etwa OH-, CO-, CN-, Cl-, Br-Gruppen tragen und auch ein- oder mehrfach ungesättigt sein können. Beispiele für solche Monocarbonsäuren sind Ameisensäure, Essigsäure, Propionsäure, Buttersäure oder Milchsäure. Des weiteren können auch aliphatische Di- und Tricarbonsäuren beim erfindungsgemäßen Verfahren verwendet werden, wobei diese Carbonsäuren auch ein- oder mehrfach ungesättigt und gegebenenfalls noch Substituenten wie etwa OH-Gruppen tragen können. Beispiele für solche Säuren sind Citronensäure, Weinsäure, Bernsteinsäure, Maleinsäure, Fumarsäure oder Äpfelsäure. Anstelle der organischen Säuren können auch acide organische Keto- oder Hydroxy-Verbindungen, wie z.B. Ascorbinsäure, Verwendung finden. Diese Calciumsalze können in wasserfreier Form, als Hydrate oder als feuchte Produkte eingesetzt werden. Besonders bevorzugt werden physiologisch verträgliche und lebensmittelrechtlich zugelassene Verbindungen eingesetzt.

Auch die Brenztraubensäure kann beim erfindungsgemäßen Verfahren wahlweise als wasserfreie Säure, als wäßrige Lösung, gelöst oder suspendiert in einem organischen Löse- oder Verdünnungsmittel zum Einsatz kommen. Darüber hinaus ist es im Rahmen der vorliegenden Erfindung auch möglich, die Brenztraubensäure in situ bzw. intermediär zu erzeugen, d.h. beispielsweise durch Umsetzung eines Alkalimetallpyruvats, wie z.B. Natrium- oder Katiumpyruvat, mit einer anorganischen Säure, wie z.B. Schwefelsäure oder Salzsäure, im Temperaturbereich von -20 bis + 90 °C, vorzugsweise -10 bis + 60°C.

Als Löse- oder Verdünnungsmittel für das erfindungsgemäße Verfahren sind Wasser und/oder organische Lösemittel, wie z.B. Alkohole (Methanol, Ethanol, Isopropanol, Cyclohexanol), Ether (Diethylether, Tetrahydrofuran, 1,4-Dioxan), Ketone (Aceton, Methylethylketon, Cyclohexanon), Ester (Methylacetat, Ethylacetat, Ethylformiat), organische Säuren (Ameisen-, Essig-, Propion-, Milch-, Brenztraubensäure), Nitrile (Acetonitril), aliphatische (Pentan, Hexan, Cyclohexan) und aromatische (Toluol) Kohlenwasserstoffe, geeignet. Die Umsetzung der organischen Calciumsalze mit Brenztraubensäure kann jedoch ohne weiteres auch in Abwesenheit von Löse- oder Verdünnungsmitteln durchgeführt werden.

Das Verhältnis von organischem Calciumsalz zu Brenztraubensäure kann in weiten Grenzen variiert werden; so kann z.B. ein molares Verhältnis im Bereich von 10:1 bis 1:20, vorzugsweise von 5:1 bis 1:10 eingesetzt werden. Als besonders vorteilhaft hat es sich jedoch erwiesen, die organischen Calciumsalze und die Brenztraubensäure in stöchiometrischen oder in annähernd stöchiometrischen Verhältnissen, z.B. in einem molaren Verhältnis von 2:1 bis 1:4, zur Umsetzung zu bringen.

Die Durchführung des erfindungsgemäßen Verfahrens ist weitgehend unproblematisch und kann nach den üblichen Methoden und in bekannten verfahrenstechnischen Apparaten wie in Knetern, Mischern, Schaufeltrocknern und Rührbehältern, erfolgen.

Auf diese Weise werden Calciumpyruvate in hoher Ausbeute (>95%) und Reinheit (>97 %) erhalten, ohne daß irgendwelche aufwendigen Reinigungsschritte erforderlich sind. Insbesondere werden mit Hilfe des erfindungsgemäßen Verfahrens neuartige Calciumpyruvate erhalten, die nicht nur sehr rein und lagerstabil sind, sondern darüber hinaus noch weitgehend wasserfrei sind und folgende Strukturformel aufweisen:

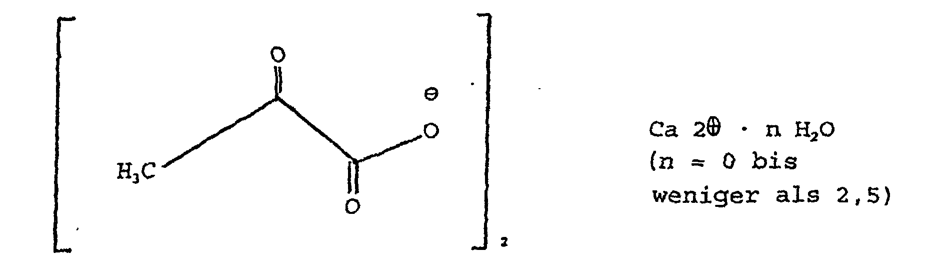

Wie die IR-spektroskopischen Untersuchungen gezeigt haben, liegen die erfindungsgemäß hergestellten Calciumpyruvate, die weniger als 2,5 Mol Kristallwasser enthalten, im wesentlichen als 2-Oxo-propionation vor.

Aufgrund der hohen Reinheit und ausgezeichneten Lagerstabilität eignen sich die erfindungsgemäß hergestellten Calciumpyruvate in hervorragender Weise als Bestandteile physiologisch verträglicher Zusammensetzungen, z.B. bei der Anwendung in der Medizin und als Nahrungsergänzungsmittel.

So können die erfindungsgemäß hergestellten Calciumpyruvate zusammen mit mindestens einer weiteren physiologisch verträglichen Substanz, die beispielsweise aus pharmazeutischen Wirkstoffen, pharmazeutischen Hilfsund Trägerstoffen, Vitaminen, Mineralstoffen, Kohlenhydraten und anderen Nahrungsergänzungsmitteln ausgewählt wird, zur Herstellung physiologisch verträglicher Zusammensetzungen eingesetzt werden.

Insbesondere können diese Calciumpyruvate zur Steigerung der Ausdauer und Kraft im Sportbereich, zur Gewichts- und Körperfettreduzierung sowie als Schutzsubstanz für Körperzellen und Gewebe (insbesondere kardiovaskuläres, hepatisches, nephrotisches, peritoneales und neuronales Gewebe) und als Substanz zur Inhibition der Radikalbildung sowie als Radikalfängersubstanz in Körperzellen und Geweben (auch Synovialgewebe) im Gesundheitsbereich, bei der Behandlung von Fettsucht und Übergewicht und als Nahrungsergänzungsmittel eingesetzt werden.

Die nachfolgenden Beispiele sollen die Erfindung näher veranschaulichen:

### Beispiel 1

Zu einer Lösung von 88 g (1 mol) reiner Brenztraubensäure (99%ig) in 400 ml Ethylacetat werden bei 20°C innerhalb 1 Stunde 81 g (0,46 mol) Calciumacetat-Monohydrat gegeben und über 18 Stunden gerührt. Schließlich wird das gebildete Calciumpyruvat abgesaugt und mit 2 x 250 ml Ethylacetat gewaschen. Die Ausbeute an Calciumpyruvat-Monohydrat beträgt 102 g (95% d.Th.).
(C₃H₃O₃)₂Ca x 1H₂O, ber.: C 31,04%, H 3,47%, Ca 17,26%; gef.: C 31,19%, H 3,58%, Ca 17,20%; Schmp. > 300°C; IR (KBr) [1/cm]: 634, 742, 832, 1185, 1354, 1402, 1643, 1713, 3195, 3480; ¹H-NMR (D₂O, 300 MHz): δ = 2,36 (s, 3H, CH₃-CO), 1,49 (s, 3H, CH₃-C(OH)₂); HPLC-Gehalt (Calciumpyruvat): 92,1% = 99,8% Calciumpyruvat-Monohydrat.

### Beispiel 2

Zu einer Lösung von 45,5 g (0,5 mol) 98,7 %iger Brenztraubensäure in 200 ml Eisessig werden 9,5 g Wasser gegeben und bei 40°C innerhalb 1 Stunde 41,8 g (0,25 mol) Calciumacetat-Semihydrat eingetragen. Das Gemisch wird bei 40°C weitere 3 Stunden gerührt. Nach Abkühlen auf 15°C wird 1 Stunde nachgerührt. Schließlich wird das Calciumpyruvat abgesaugt, mit 2 x 100 ml Ethylacetat gewaschen und bei 50°C und 15 mbar getrocknet. Die Ausbeute an Calciumpyruvat-Monohydrat beträgt 55 g (95% d.Th.).

### Beispiel 3

Bei einer Temperatur von 15 bis 20 °C werden zu einer Suspension von 110 g (1 mol) Natriumpyruvat in 200 ml Ethylacetat über eine Zeit von 45 Minuten 64,3 g (0,49 mol) 70 %ige Schwefelsäure zugetropft. Nach 3 Stunden wird das ausgefallene Natriumsulfat abgesaugt und mit 2 x 40 ml Ethylacetat gewaschen. Zum Filtrat werden 250 g konzentrierte Essigsäure gegeben. Das Gemisch wird auf 35°C erwärmt. Innerhalb von 30 Minuten werden 80,2 g (0,48 mol) Calciumacetat-Semihydrat eingetragen. Die Nachrührzeit der dünnflüssigen Suspension beträgt 3 Stunden. Schließlich wird das Calciumpyruvat abgesaugt und mit 2 x 100 ml Ethylacetat gewaschen. Das Produkt wird bei 50°C im Vakuumtrockenschrank bis zur Gewichtskonstanz getrocknet. Die Ausbeute an Calciumpyruvat-Monohydrat beträgt 107 g (96% d.Th.).

### Beipiel 4

In einem Laborkneter werden 88 g (1 mol) Brenztraubensäure (99%) zu 84 g (0,5 mol) Calciumacetat-Semihydrat bei 20 °C über einen Zeitraum von 30 Minuten gegeben und über 2 Stunden geknetet. Anschließend wird das essigsäure-feuchte Calciumpyruvatim Vakuumtrockenschrank bei 50°C und 12 mm Hg getrocknet. Die Ausbeute an Calciumpyruvat-Semihydrat ist nahezu quantitativ (>99% d.Th.).

### Beispiel 5

Zu einer Lösung von 45,5 g (0,5 mol) 98,7 %iger Brenztraubensäure in 200 ml Eisessig werden 20 g Wasser gegeben und bei 40 °C innerhalb 1 Stunde 32,5 g (0,25 mol) Calciumformiat eingetragen. Bei dieser Temperatur wird 3 Stunden gerührt. Nach Abkühlen auf 15 °C wird 1 Stunde nachgerührt. Schließlich wird das gebildete Calciumpyruvat abgesaugt, mit 2 x 100 ml Ethylacetat gewaschen und bei 50 °C und 15 mbar getrocknet. Die Ausbeute an Calciumpyruvat-Trihydrat beträgt 65 g (97 % d. Th.),
(C₃H₃O₃)₂ Ca x 3 H₂O, ber.: C 26,87%, H 4,51 %, Ca 14,94 %; gef.: C 26,77%, H 4,53 %, Ca 14,70 %; Schmp. > 300°C; IR (KBr) [1/cm]: 668, 789, 862, 934, 965, 1142, 1182, 1408, 1610, 3430; ¹H-NMR (D₂O, 300 MHz): δ = 2,36 (s, 3H, CH₃-CO), 1,49 (s, 3H, CH₃-C(OH)₂); HPLC-Gehalt (Calciumpyruvat): 79,4% = 99,4 % Calciumpyruvat-Trihydrat.

### Beispiel 6 (Vergleich)

### Nacharbeitung des Verfahrens gemäß Monatshefte 6, 467-476 (1885) (K. Jowanowitsch)

Ein Gemisch aus 40 g Glycerin und 32 g Weinsäure wird auf 140 °C erwärmt, bis kein Wasserdampf mehr entweicht. Dann wird das Gemisch auf 260 °C erhitzt, wobei das unter Gasentwicklung übergehende Destillat fraktioniert aufgefangen wird. Als erste Fraktion wird eine dünnflüssige Emulsion erhalten, aus der sich 0,2 g eines kristallinen Feststoffes abscheiden. Bei diesem Feststoff handelt es sich laut NMR-, IR- und GC-MS-Analysen um das Brenztraubensäureglycid. Die Gesamtmenge wird in 5 ml Wasser gelöst und nach Zugabe von 80 mg Calciumcarbonat 30 Minuten zum Sieden erhitzt. Die wäßrige Lösung wird nach Abtrennung des überschüssigen Calciumcarbonats HPLC-chromatographisch analysiert, wobei jedoch kein Calciumpyruvat nachweisbar ist.

## Patentansprüche

1. Verfahren zur Herstellung von Calciumpyruvaten.
**dadurch gekennzeichnet,**
**daß** man Calciumsalze von organischen Säuren oder aciden organischen Keto- oder Hydroxy-Verbindungen mit Brenztraubensäure im Temperaturbereich von -20 bis + 120°C umsetzt und die gebildeten Calciumpyruvate gewinnt.

2. Verfahren nach Anspruch 1,
**dadurch gekennzeichnet,**
**daß** man als organische Säure eine aliphatische Monocarbonsäure einsetzt.

3. Verfahren nach Anspruch 1,
**dadurch gekennzeichnet,**
**daß** man als organische Säure eine aliphatische Di- oder Tricarbonsäure verwendet.

4. Verfahren nach Anspruch 1,
**dadurch gekennzeichnet,**
**daß** man als acide organische Keto- oder Hydroxyverbindung Ascorbinsäure einsetzt.

5. Verfahren nach einem der Ansprüche 1 bis 4,
**dadurch gekennzeichnet,**
**daß** die Brenztraubensäure in situ erzeugt worden ist.

6. Verfahren nach Anspruch 5,
**dadurch gekennzeichnet,**
**daß** die Brenztraubensäure durch Umsetzung eines Alkali-metallpyruvats mit einer anorganischen Säure, wie z.B. Schwefel- oder Salzsäure, intermediär gebildet worden ist.

7. Verfahren nach einem der Ansprüche 1 bis 6,
**dadurch gekennzeichnet,**
**daß** man die Umsetzung in einem Temperaturbereich von 10 bis 60°C vornimmt.

8. Verfahren nach einem der Ansprüche 1 bis 7,
**dadurch gekennzeichnet,**
**daß** man die Umsetzung in Gegenwart eines Löse- oder Verdünnungsmittels durchführt.

9. Verfahren nach Anspruch 8,
**dadurch gekennzeichnet,**
**daß** man als Löse- oder Verdünnungsmittel ein organisches Lösemittel und/oder Wasser verwendet.

10. Verfahren nach Anspruch 9,
**dadurch gekennzeichnet,**
**daß** man das organische Lösemittel aus Alkoholen, Ethern, Ketonen, Estern, organischen Säuren, Nitrilen, aliphatischen und aromatischen Kohlenwasserstoffe und Gemischen davon auswählt.

11. Verfahren nach einem der Ansprüche 1 bis 9,
**dadurch gekennzeichnet,**
**daß** man die Brenztraubensäure und die organischen Calciumsalze in einem molaren Verhältnis von 2:1 bis 1:4 zur Umsetzung bringt.

12. Verfahren nach einem der Ansprüche 1 bis 11,
**dadurch gekennzeichnet,**
**daß** man die gewonnenen Calciumpyruvate zur Herstellung einer physiologisch verträglichen Zusammensetzung verwendet.

13. Calciumpyruvate der allgemeinen Formel

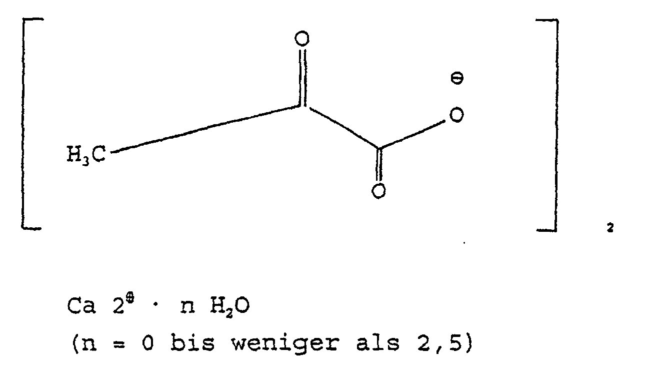

14. Physiologisch verträgliche Zusammensetzungen, die Calciumpyruvate nach Anspruch 13 zusammen mit mindestens einer weiteren physiologisch verträglichen Substanz ausgewählt aus pharmazeutischen Wirkstoffen, pharmazeutischen Hilfs- und Trägerstoffen, Vitaminen, Mineralstoffen, Kohlenhydraten und anderen Nahrungsergänzungsmitteln enthalten.

15. Verwendung der Calcium-Pyruvate nach Anspruch 13 zur Herstellung eines Mittels für die Steigerung der Ausdauer und Kraft im Sportbereich, zur Gewichts- und Körperfettreduzierung, als Schutzsubstanz für Körperzellen und Gewebe, als Substanz zur Inhibition der Radikalbildung sowie als Radikalfängersubstanz in Körperzellen und Geweben, im Gesundheitsbereich, bei der Behandlung von Fettsucht und Übergewicht und als Nahrungsergänzungsmittel.

## Claims

1. A method of producing calcium pyruvates,
**characterised in that**
calcium salts of organic acids or acidic organic keto or hydroxy compounds are reacted with pyruvic acid at a temperature in the range from -20 to +120°C, and the calcium pyruvates thus formed are obtained.

2. The method of claim 1,
**characterised in that**
an aliphatic monocarboxylic acid serves as organic acid.

3. The method of claim 1,
**characterised in that**
an aliphatic di- or tricarboxylic acid serves as organic acid.

4. The method of claim 1,
**characterised in that**
ascorbic acid serves as acidic organic keto or hydroxy compound.

5. The method according to one of claims 1 to 4,
**characterised in that**
the pyruvic acid is produced in situ.

6. The method of claim 5,
**characterised in that**
the pyruvic acid is formed as an intermediate by reacting an alkali-metal pyruvate with an inorganic acid such as sulphuric or hydrochloric acid.

7. The method according to one of claims 1 to 6,
**characterised in that**
the reaction is carried out at a temperature in the range from 10 to 60°C.

8. The method according to one of claims 1 to 7,
**characterised in that**
the reaction is carried out in the presence of a solvent or diluent.

9. The method of claim 8,
**characterised in that**
an organic solvent and/or water is used as solvent or diluent.

10. The method of claim 9,
**characterised in that**
the organic solvent is selected from the group comprising alcohols, ethers, ketones, esters, organic acids, nitriles, aliphatic and aromatic hydrocarbons and mixtures thereof

11. The method according to one of claims 1 to 9,
**characterised in that**
the pyruvic acid and the organic calcium salts are made to react in a molar ratio of 2:1 to 1:4..

12. The method according to one of claims 1 to 11,
**characterised in that**
the calcium pyruvates obtained are used to produce a physiologically compatible composition.

13. Calcium pyruvates of the general formula

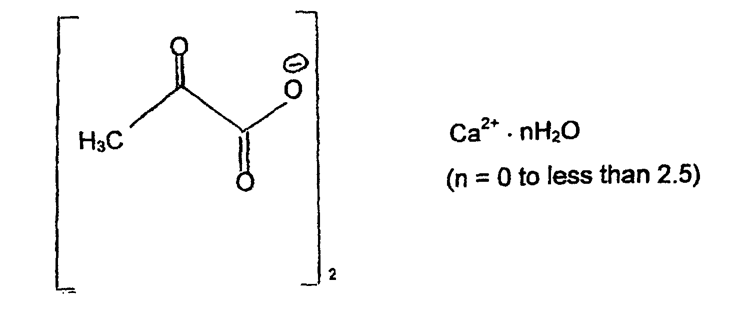

14. Physiologically compatible compositions which contain calcium pyruvates according to claim 13, together with at least one other physiologically compatible substance selected from the group comprising pharmaceutical active ingredients, pharmaceutical adjuvants and carriers, vitamins, mineral substances, carbohydrates and other food supplements.

15. Use of the calcium pyruvates according to claim 13 for enhancing stamina and vigour in the field of sport, for reducing weight and body-fat, as a protective substance for body cells and tissues and as a substance which inhibits the formation of free radicals and which scavenges free radicals in body cells and tissues in the field of health care, and also for treating obesity and weight problems and as a food supplement.

## Revendications

1. Procédé de préparation de pyruvates de calcium **caractérisé en ce que** l'on fait réagir des sels de calcium d'acides organiques ou de composés cétoniques ou hydroxylés organiques acides avec l'acide pyruvique dans le domaine de température de -20 à +120°C et on obtient les pyruvates de calcium formés.

2. Procédé selon la revendication 1 **caractérisé en ce que** l'on utilise comme acide organique un acide monocarboxylique aliphatique.

3. Procédé selon la revendication 1 **caractérisé en ce que** l'on utilise comme acide organique un acide di- ou tricarboxylique aliphatique.

4. Procédé selon la revendication 1 **caractérisé en ce que** l'on utilise comme composé cétonique ou hydroxylé organique acide l'acide ascorbique.

5. Procédé selon l'une des revendications 1 à 4 **caractérisé en ce que** l'acide pyruvique a été produit in situ.

6. Procédé selon la revendication 5 **caractérisé en ce que** l'acide pyruvique a été produit de manière intermédiaire par réaction d'un pyruvate de métal alcalin avec un acide inorganique comme l'acide sulfurique ou l'acide chlorhydrique, par exemple.

7. Procédé selon l'une des revendications 1 à 6 **caractérisé en ce que** l'on conduit la réaction dans un domaine de température de 10 à 60°C.

8. Procédé selon l'une des revendications 1 à 7 **caractérisé en ce que** l'on conduit la réaction en présence d'un solvant ou diluant.

9. Procédé selon la revendication 8 **caractérisé en ce que** l'on utilise un solvant organique et/ou l'eau comme solvant ou diluant.

10. Procédé selon la revendication 9 **caractérisé en ce que** l'on choisit le solvant organique parmi les alcools, les éthers, les cétones, les esters, les acides organiques, les nitriles, les hydrocarbures aliphatiques et aromatiques et leurs mélanges.

11. Procédé selon l'une des revendications 1 à 9 **caractérisé en ce que** l'on fait réagir l'acide pyruvique et les sels de calcium organiques dans un rapport molaire de 2:1 à 1:4.

12. Procédé selon l'une des revendications 1 à 11 **caractérisé en ce que** l'on utilise les pyruvates de calcium obtenus pour la préparation d'une composition physiologiquement acceptable.

13. Pyruvates de calcium de formule générale

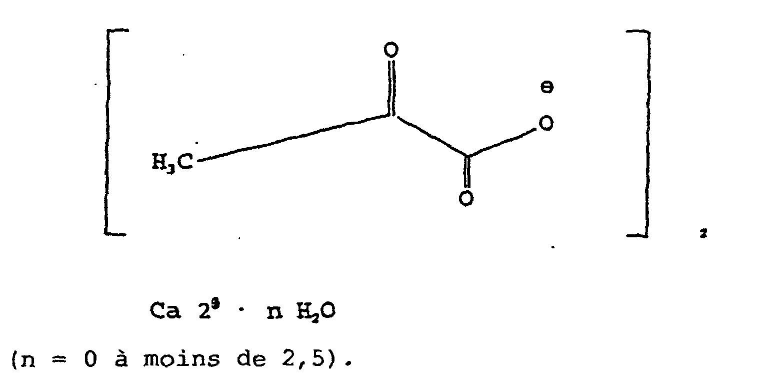

14. Compositions physiologiquement acceptables qui contiennent des pyruvates de calcium selon la revendication 13 ainsi qu'au moins une autre substance physiologiquement acceptable choisie parmi les principes actifs pharmaceutiques, les adjuvants et supports pharmaceutiques, les vitamines, les substances minérales, les glucides et d'autres compléments alimentaires.

15. Utilisation des pyruvates de calcium selon la revendication 13 pour la préparation d'un agent pour augmenter l'endurance et la vigueur dans le domaine du sport, pour réduire le poids et les graisses corporelles, comme substance protectrice pour les cellules corporelles et les tissus, comme substance pour inhiber la formation de radicaux ainsi que comme substance piégeant les radicaux dans les cellules corporelles et les tissus, dans le domaine de la santé, lors du traitement de l'adipose et du surpoids et comme complément alimentaire.
